# EUROPÄISCHE PATENTSCHRIFT

(11) **EP 0 570 675 B1**
(45) Veröffentlichungstag und Bekanntmachung des Hinweises auf die Patenterteilung: **18.10.1995**
(21) Anmeldenummer: 93104077.8
(22) Anmeldetag: 12.03.1993
(51) Int. Cl.: B25B 7/00, B25B 7/12, A61B 17/28

(54) **Zange zum Fassen und Halten von Gewebe oder dergleichen**
Forceps for gripping and holding tissue or the like
Pince pour la préhension et le maintien de tissus

(30) Priorität: 22.05.1992 DE 4216971
(43) Veröffentlichungstag der Anmeldung: 24.11.1993
(73) Patentinhaber: Richard Wolf GmbH, 75438 Knittlingen (DE)
(72) Erfinder: Boebel, Manfred, W-7136 Oetisheim (DE)
(74) Vertreter: Wilcken, Thomas, Dipl.-Ing.

(56) Entgegenhaltungen:
- GB-A- 2 208 201
- US-A- 4 944 739
- US-A- 5 009 661
- DATABASE WPI Week 8931, Derwent Publications Ltd., London, GB; AN 89-225985 & SU-A-1 437 207 (YURKOV)

## Beschreibung

Die Erfindung geht aus von einer Zange mit Zwei Maulteilen Zum Fassen und Halten von Gewebe oder dergleichen, bei der mindestens ein Maulteil relativ zum anderen durch Verstellung einer Betätigungsstange mittels einer Handhabe verschwenkbar ist und die Schließstellung der Maulteile und der Handhabe durch eine Arretierung fixierbar ist.

Zangen dieser Art müssen so konstruiert werden, daß sie das Gewebe absolut zuverlässig halten und fixieren können. Es sind bereits Gewbezangen bzw. -klemmen zum Unterbinden und Ligieren von gefäßversorgten Organen bzw. Geweberegionen bekannt, welche eine sichere Fixierung des erfaßten Gewebes durch eine speziell entwickelte Rastmechanik (DE 39 31 577 A) gewährleisten. Bei diesem Rastmechanismus rastet bei derartigen Zangen bzw. Klemmen die Mechanik nach Überwindung eines Druckpunktes zuverlässig ein und fixiert diese sicher. Die Freigabe der Fixierung erfolgt über einen weiteren Druckpunkt.

Es ist bedeutungslos, wenn derartige Zangen das erfaßte Gewebe traumatisieren, sofern dieser Gewebeteil ohnehin aus dem Körper entfernt wird. Eine Traumatisierung ist allerdings dann nicht zulässig, wenn beispielsweise Organe oder Gewebeteile, die ihre Aufgabe weiterhin im Körper erfüllen sollen und lediglich zur Manipulation erfaßt bzw. fixiert werden sollen, in dem Maul einer Gewebefaßzange festgelegt werden. In diesem Fall muß ein weicher, atraumatischer Zugriff des Gewebes erfolgen.

Bei den herkömmlichen Zangen ist es zwar grundsätzlich möglich, durch die Handkraft am Griff des Instrumentes diese so zu dosieren, daß das Gewebe nicht traumatisiert wird. Der Nachteil liegt jedoch darin, daß bei derartiger Handhabung des Instrumentes eine Fixierung der jeweils erreichten Maulstellung mit einem Rastmechanismus nicht möglich ist und somit kein zuverlässiges und sicheres Halten des Gewebes gewährleistet ist.

Die Aufgabe der Erfindung liegt darin, eine gattungsgemäße Zange so auszubilden, daß die auf den zwischen den beiden Zangenmaulteilen befindlichen Gewebeabschnitt oder dergleichen wirkende Schließkraft verändert und fixiert werden kann, um bei Bedarf eine Traumatisierung von Gewebe zu verhindern. Im übrigen soll die Zange alle auch schon zuvor genannten Anforderungen erfüllen, insbesondere in bezug auf eine absolut sichere Funktion.

Die Aufgabe wird erfindungsgemäß dadurch gelöst, daß bei der einleitend erwähnten Zange die Schließkraft der Zangenmaulteile auf unterschiedliche Werte einstellbar ist, wobei die über die Handhabe auf die Betätigungsstange ausgeübte Kraft bei Einstellung der maximalen Schließkraft direkt und bei Einstellung einer anderen niedrigeren Schließkraft mittelbar über mindestens eine Feder auf das bewegliche Maulteil übertragen wird.

Durch diese erfindungsgemäße Lösung kann einerseits, wie bereits aus dem Stand der Technik bekannt, die Zange durch eine Arretierung fixiert werden, und andererseits sind zusätzlich verschiedene Schließkräfte stufenweise definiert, so daß das Gewebe nicht traumatisiert wird.

Außerdem kann sowohl die maximale Schließkraft - etwa zum Fassen von Gewebe, bei dem es unbedeutend ist, ob es traumatisiert wird oder nicht - eingestellt werden, als auch andere kleinere Schließkräfte - etwa zum Fassen von Gewebe, welches auf Keinen Fall traumatisiert werden darf - und zwar je nach Empfindlichkeit des zu fassenden Gewebes.

Die Betätigungsstange der Zange besteht aus einem distalen ersten Stangenteil und einem von diesem aus proximalwärts verlaufenden zweiten Stangenteil, wobei der erste direkt mit dem beweglichen Maulteil in Verbindung steht. Zwecks Einstellung verschiedener Schließkräfte ist der zweite Stangenteil relativ zum ersten verdrehbar und ist mit im axialen Abstand zueinander sowie in Winkeln zueinander versetzt angeordneten Mitnehmern ausgestattet, die mit Kulissen zusammenarbeiten, so daß bei einer vorgegebenen Drehstellung des zweiten Stangenteiles, welche die Schließkraft vorgibt, nur der für diese Schließkraft erforderliche Mitnehmer mit der diesem zugeordneten Kulisse in Eingriff steht. Die definierte Betätigungskraft kann somit unmittelbar oder mittelbar über Druckfedern vom zweiten auf den ersten Stangenteil übertragen werden, was davon abhängt, welcher der Mitnehmer mit der ihm angeordneten Kulisse in Eingriff gebracht ist. Es versteht sich, daß die Anzahl der für die einstellbaren Stufen notwendigen Bauteile - wie Mitnehmer, Kulissen und in Reihe geschaltete Federn - von der Anzahl der gewünschten Schließkraftstufen abhängt.

In den Kulissen sind Ausnehmungen vorgesehen, die für einen freien axialen Durchgang von nicht in Schaltstellung befindlichen Mitnehmern sorgen. Zudem bringen die Druckfedern die Kulissenteile gegen vorgesehene Anschläge bzw. Ringschultern zur Anlage. Derartige Ringnuten sichern in Ruhestellung befindliche Kulissen gegen Bewegung in proximaler Richtung.

Weiterhin weist die Betätigungsstange proximalseitig einen Schaltring auf, der mit einer relativ zu einem feststehenden Teil verdrehbaren Feder ausgestattet ist, und diese besitzt eine nach innen gerichtete Nase, die je nach Schaltstellung in jeweils eine von mehreren Ausnehmungen am erwähnten feststehenden Teil eingreift und so die Drehstellungen des zweiten Stangenteils fixiert, um unterschiedliche Schließkräfte gezielt einstellen zu können. Die verdrehbare Feder kann derart ausgestattet werden, daß sie durch einen Stift unterbrochen und am Schaltring festgelegt wird. Diese Maßnahme verhindert eine Verstellung bzw. ein Verdrehen der geteilten Feder innerhalb einer zwischen dem Schaltring und der Feder befindlichen Ringnut. Der Schaltring und folglich auch der proximalseitige Stangenteil können hierdurch nur in definierter Stellung fixiert werden.

Die Übertragung der Drehbewegung des Schaltringes auf den proximalseitigen Stangenteil erfolgt über eine Zylinderhülse. Beide Betätigungsstangenteile sind mit einem Stift axial beweglich geführt und gegen Radialbewegung gesichert.

Die Erfindung wird nachfolgend anhand eines in der anliegenden Zeichnung dargestellten Ausführungsbeispieles näher erläutert. Es zeigen:

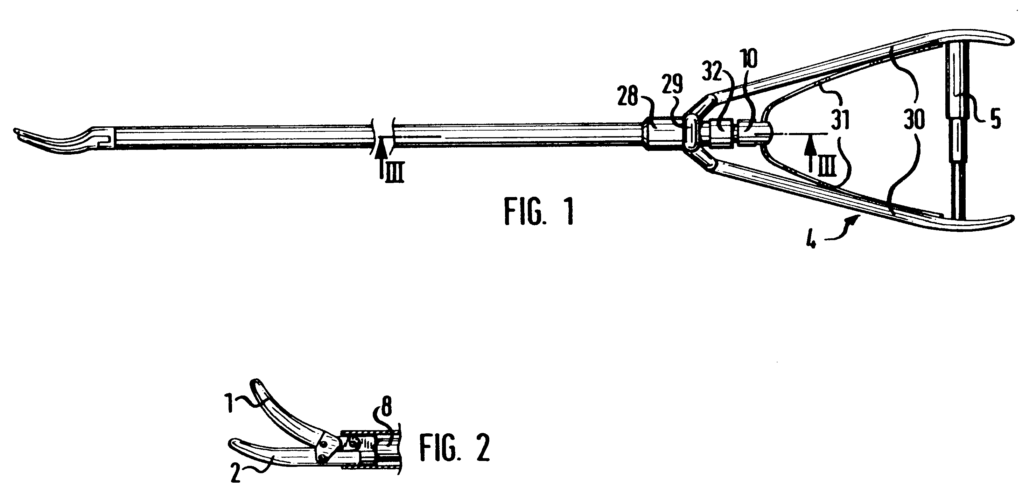

- Figur 1: eine Seitenansicht der erfindungsgemäßen Zange,
- Figur 2: einen teilweisen Längsschnitt durch den distalen Endbereich der Zange mit offener Maulteilstellung,
- Figur 3: einen Schnitt entsprechend der Linie III - III durch das proximale Zangenende nach Figur 1 mit der in größerem Maßstab dargestellten Vorrichtung zur Umschaltung der Schließkraft,
- Figur 4: einen Schnitt gemäß Linie IV - IV in Figur 3 in vergrößertem Maßstab und
- Figur 5: einen Schnitt gemäß Linie V - V in Figur 3 in vergrößertem Maßstab.

Die erfindungsgemäße Gewebezange hat zwei Maulteile 1 und 2 zum Fassen und Halten des Gewebes am distalen Ende, eine mit diesen verbundene Betätigungsstange 3 und eine Handhabe 4 mit integrierfern Rastmechanismus bzw. Arretierung 5 am proximalem Ende, die für eine Fixierung der Schließstellung der Maulteile 1 und 2 sorgt. Ein Maulteil 1 ist relativ zum anderen 2 durch axiale Verstellung der Betätigungsstange 3 mittels der Handhabe 4 verschwenkbar.

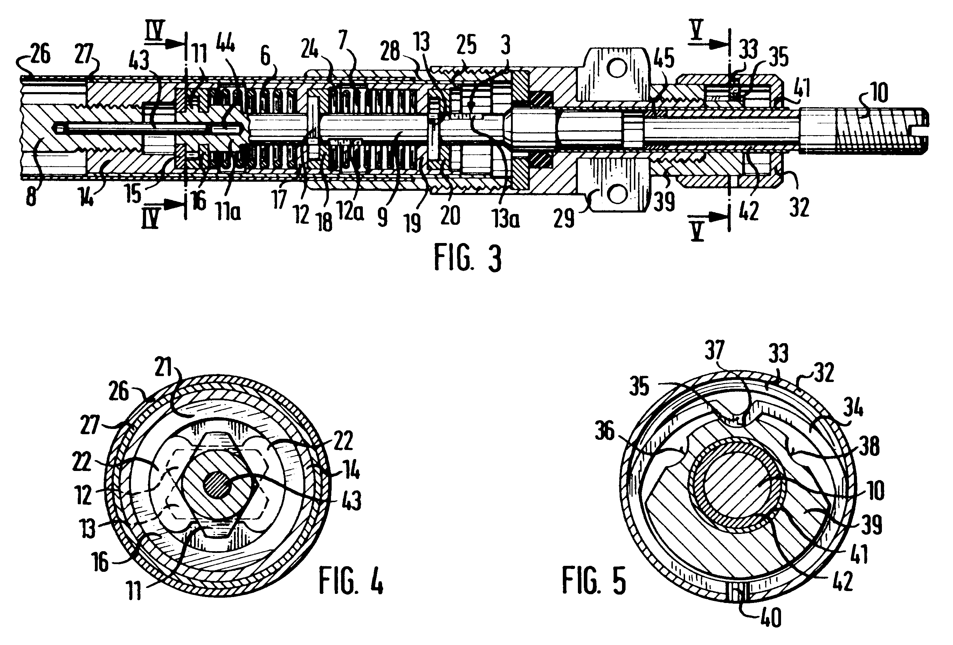

Die auf die beiden Zangenmaulteile 1 und 2 mit der Betätigungsstange 3 übertragbare Schließkraft kann beispielsweise in drei definierten Stufen einstellbar sein. Figur 3 zeigt ein solches Ausführungsbeispiel mit entsprechenden Bauteilen. Hier ist die Vorrichtung zum Umschaltung der Schließkraft dargestellt.

Es sind zwei Druckfedern 6 und 7 vorgesehen, welche die über die Handhabe 4 auf die Betätigungsstange 3 ausgeübte Kraft bei Einstellung niedriger Schließkräfte mittelbar auf das bewegliche Maulteil 1 übertragen. Soll die maximale Schließkraft übertragen werden, so erfolgt dieses unmittelbar, also nicht über zwischengeschaltete Druckfedern, wie es noch später erläutert wird.

Die Betätigungsstange 3 besteht aus einem distalen ersten Stangenteil 8 und einem von diesem aus proximalwärts verlaufenden zweiten Stangenteil 9 und und einem dritten Stangenteil 10. Der distale Teil 8 ist mit dem schwenkbeweglichen Zangenmaulteil 1 direkt verbunden, zum Beispiel über ein Gelenk. Der proximalwärts verlaufende zweite Stangenteil 9 ist so ausgeführt, daß er relativ zum ersten 8 drehbeweglich ist. Weiterhin ist der zweite Stangenteil 9 mit im axialen Abstand zueinander sowie in Winkeln zueinander versetzt angeordneten Mitnehmern 11,12 und 13 ausgestattet, die mit axial beweglichen Kulissen 14, 15, 16, 17, 18, 19 und 20 zusammenarbeiten.

Soll die maximale Schließkraft übertragen werden, so steht bei der Drehstellung des zweiten Stangenteiles 9 gemäß Figur 3 nur der Mitnehmer 11 mit der mit dem ersten Stangenteil 8 fest verbundenen ersten Kulisse 14, 15 und 16 in Eingriff. Bei stufenweise reduzierter Schließkraft steht zunächst der Mitnehmer 12 und im Anschluß daran der Mitnehmer 13 mit den jeweils zugeordneten Kulissen 17, 18 in Eingriff. Dann wird über die aktive Kulisse und die Druckfeder 6 die Betätigungskraft auf den ersten Stangenteil 8 übertragen. Die Kulissenteile 14 bis 20 sind durch die Druckfedern 6 und 7 in proximaler Richtung federvorgespannt. Es versteht sich, daß sich die Anzahl der Bauelemente für die stufenweise Schließkrafteinstellung - wie Druckfedern, Mitnehmer und Kulissen -nach der Anzahl der vorgesehenen Schließkraftstufen richtet. In dem hier gezeigten Beispiel sind drei Stufen vorgesehen.

Figur 3 zeigt eine Position, bei welcher die maximale Schließkraft eingestellt ist. Dieses wird dadurch bewirkt, daß der distalseitige Mitnehmer 11 in der Kulisse 14, 15 und 16 lösbar festgelegt ist und somit die auf den proximalwärts verlaufenden zweiten und dritten Stangenteil 9, 10 ausgeübte Kraft auf direktem Wege über die Kulissenteile 14, 15 auf den ersten Stangenteil 8 übertragen wird, während der mittlere 12 und der proximalseitige Mitnehmer 13 jeweils ungehindert die zugehörige mittlere 17,18 und proximalseitige Kulisse 19, 20 frei durchlaufen können.

Figur 4 verdeutlicht nochmals die zuvor geschilderte Einstellposition. In der Schnittdarstellung ist erkennbar, daß der distale Mitnehmer 11 an dem Steg 21 zwischen den beiden in den Kulissenteilen 15 und 16 befindlichen ovalen Ausnehmungen 22 angreift, so daß die Kraftübertragung bei axialem Verschieben des zweiten und dritten Stangenteiles 9,10 auf den ersten Stangenteil 8 sowohl in distaler als auch in proximaler Richtung erfolgen kann. Die Ausnehmungen 22 dienen einem freien axialen Durchgang der jeweils nicht aktiven Mitnehmer. In diesem Fall sind es die Mitnehmer 12 und 13.

Die Druckfedern 6, 7 bringen die Kulissenteile 18,20 gegen Ringschultern 24 und 25 zur Anlage, und zwar derart, daß die Ringschultern 24, 25 die in Ruhestellung befindlichen Kulissenteile gegen proximalwärtige Bewegung sichern. Die Ringschultern 24,25 sind in der Vorrichtung derart angeordnet, daß sie in einer koaxial im Außenschaft 26 angeordneten Zylinderhülse 27 unlösbar festgelegt sind. Diese sind wiederum mit ihrem proximalseitigen Ende in dem Gehäuseteil 28 festgelegt, das beispielsweise über eine Schraubverbindung mit dem proximalen Gehäuseteil 29 verbunden ist, an dessen proximalen Ende Handgriffe 30 schwenkbeweglich festgelegt sind.

Zwischen den beiden Handgriffen 30 befinden sich federnde Bügel 31 für die Bewegungsübertragung, die beim Zusammendrücken der beiden Handgriffe 30 die Betätigungsstange 3 so verstellen, daß die Handhabe 4 die Kraft von den Handgriffen 30 ausgehend über die Bügel 31 auf den proximalseitigen Stangenteil 10 überträgt, so daß schließlich die Stangenteile 8, 9 und 10 distalwärts axial verschoben werden. Im übrigen stellen die Bügel 31 die Handhabe 4 und die Betätigungsstange 3 nach dem Lösen der Arretierung 5 selbsttätig in die Ausgangsposition zurück.

Zur Einstellung unterschiedlicher Schließkräfte ist an dem proximalseitigen Stangenteil 10 ein von Hand verdrehbarer hohlzylindrisch ausgebildeter Schaltring 32 vorgesehen, über welchen eine Verdrehung des Stangenteiles 9 ermöglicht wird.

Der Schaltring 32 weist eine relativ zum feststehenden Teil 39 verdrehbare Ringfeder 34 auf. Diese greift mit einer nach innen gerichteten Nase 35 je nach einzustellender Schließkraft in eine der vorgesehenen Ausnehmungen 36, 37 oder 38 des feststehenden Teiles 39 ein. Durch diese Konstruktion können unterschiedliche Einstellungen gezielt und sicher vorgenommen werden, da die jeweils eingestellte Position des Stangenteiles 9 durch das Einrasten der Nase 35 in eine der Ausnehmungen 36, 37, 38 fühlbar ist.

Figur 4 und Figur 5 zeigen das Zusammenwirken der einzelnen Bauteile. Wird mittels des Schaltringes 32 über die Zylinderhülse 41 der Stangenteil 9 so weit verdreht, bis die Ringfeder 34 mit ihrer Nase 35 aus der Ausnehmung 36 herausgeführt wird und bei weiterem Verdrehen in die Ausnehmung 37 eingreift, so befindet sich der mittlere Mitnehmer 12 mit den entsprechenden Kulissen 17 und 18 im Eingriff, während sich sowohl der distale 11 als auch der proximale Mitnehmer 13 mit den zugehörigen Kulissen 14, 15, 16 und 19, 20 außer Eingriff befinden. In dieser Position wird die Schließkraft durch die Druckfeder 6 bestimmt. In dieser zweiten Einstellung ist die Schließkraft geringer. Bei weiterem Verdrehen des Schaltringes 32 um den gleichen Winkel wird die dritte Position erreicht, bei der die Nase 35 in die Ausnehmung 38 und der Mitnehmer 13 in die Kulisse 19,20 greift, so daß bei Betätigung der Zange die beiden dann in Reihe geschalteten Federn 6,7 die nochmals reduzierte Schließkraft auf den Stangenteil 8 und das Maulteil 1 übertragen.

Die Übertragung der Drehbewegung des Schaltringes 32 auf den verdrehbaren Stangenteil 9, 10 erfolgt über die Zylinderhülse 41, welche an ihren Enden mit den Teilen 9 und 32 unlösbar sowie mit der Zylinderhülse 42 verbunden ist. Um die Stange 3 proximalwärts zurückziehen zu können, liegt die mit dem Stangenteil 10 verbundene Zylinderhülse 45 am distalen Ende der Zylinderhülse 42 an.

Am distalen Ende des Stangenteiles 9 befindet sich eine in der Stirn-Seite zentral verlaufende Bohrung 44 zur Aufnahme eines Stiftes 43, der zentrisch im Stangenteil 8 befestigt ist und mit dem die Stangenteile 8 und 9 axial beweglich verbunden und aufgrund der dadurch erreichten Zentrierung gegen Radialbewegung gesichert ist.

Um zu verhindern, daß nach Einstellung einer ausgewählten Schließkraft und bei Verstellung der Betätigungsstange die gegenseitige Anlage der für diese Schließkraft aktivierten Einheit aus Mitnehmer und Kulissenteilen aufgehoben werden könnte, und um hierdurch mögliche Funktionsstörungen zu vermeiden, wird die aus der Ruhestellung heraus distalwärts verstellte Betätigungsstange gegen Verdrehen gesichert. Somit ist es nicht möglich, während der Betätigung der Zange durch Verdrehen des Schaltringes 32 die Betätigungsstange zu verdrehen.

Zu diesem Zweck sind gemäß Figur 3 auf der Betätigungsstange 3 Vorsprünge 11a,12a und 13a vorgesehen, von denen jeweils einer jedem Mitnehmer 11,12 und 13 mit einem etwa der Kulissenstärke entsprechenden Abstand proximalseitig zugeordnet ist und bei distalwärtiger Verstellung der Betätigungsstange in drehschlüssigen Eingriff mit der ihm zugeordneten Ausnehmung 22 gelangt.

Treten also beispielsweise die Mitnehmer 12,13 durch axiales Verschieben der Betätigungsstange 3 aus den Kulissenteilen 17,18;19,20 hervor, so gelangen die Vorsprünge 12a,13a in die zugehörige Ausnehmung 20, wodurch die Betätigungsstange gegen Verdrehen gesichert wird.

Um die Betriebssicherheit und Funktionsfähigkeit der erfindungsgemäß ausgebildeten Zange zu gewährleisten, sollten die Kulissenteile 14,17 und 19 zweckmäßigerweise gegen radiales Verdrehen gesichert werden, und zwar beispielsweise durch jeweils einen in eine axial verlaufende Nut eingreifenden Stift. Weiterhin sollen die Kulissenteile 14,15,16 und 17,18 sowie 19,20 unlösbar miteinander festgelegt sein, um dadurch zu gewährleisten, daß die komplementär ausgebildeten Ausnehmungen 22 immer die gleiche Stellung zueinander einnehmen, also während des Betriebes nicht gegeneinander verdreht werden können. Sonst würde nämlich nicht die Möglichkeit bestehen, daß die nicht im Eingriff mit einer Kulisse stehenden Vorsprünge die außer Eingriff stehenden Kulissenteile frei durchlaufen können.

## Patentansprüche

1. Zange mit zwei Maulteilen (1,2) zum Fassen und Halten von Gewebe oder dergleichen, bei der mindestens ein Maulteil (1) relativ zum anderen (2) durch Verstellung einer Betätigungsstange (3) mittels einer Handhabe (4) verschwenkbar ist und die Schließstellung der Maulteile (1,2) und der Handhabe (4) durch eine Arretierung (5) fixierbar ist, dadurch gekennzeichnet, daß die Schließkraft der Zangenmaulteile (1,2) auf unterschiedliche Werte einstellbar ist, wobei die über die Handhabe (4) auf die Betätigungsstange (3) ausgeübte Kraft bei Einstellung der maximalen Schließkraft direkt und bei Einstellung einer anderen niedrigeren Schließkraft mittelbar über mindestens eine Feder (6,7) auf das bewegliche Maulteil (1) übertragen wird.

2. Zange nach Anspruch 1, dadurch gekennzeichnet, daß die Betätigungsstange (3) aus einem distalen ersten Stangenteil (8) einem von diesem aus proximalwärts verlaufenden zweiten und dritten Stangenteil (9,10) besteht, daß der erste Stangenteil (8) direkt mit dem beweglichen Maulteil (1) und der dritte Stangenteil (10) über Federbügel (31) mit der Handhabe (4) in Verbindung steht, daß der zweite Stangenteil (9) relativ zum ersten (8) zwecks Einstellung der Schließkraftstufen verdrehbar und mit im axialen Abstand zueinander sowie in Winkeln zueinander versetzt angeordneten Mitnehmern (11,12,13) versehen ist, die mit axial beweglichen Kulissen (14,15,16;17,18;19,20) zusammenarbeiten, derart, daß bei einer vorgegebenen Drehstellung des zweiten Stangenteiles (9,10) nur ein Mitnehmer (11) mit einer mit dem ersten Stangenteil (8) fest verbundenen ersten Kulisse (14,15,16) in Eingriff ist, während bei einer anderen vorgegebenen Drehstellung ein anderer Mitnehmer (12 oder 13) mit der diesem zugeordneten Kulisse (17,18 oder 19,20) in Eingriff steht und die Betätigungskraft über diese Kulisse und mindestens eine Druckfeder (6,7) auf den ersten Stangenteil (8) überträgt.

3. Zange nach Anspruch 2, dadurch gekennzeichnet, daß die Anzahl der Mitnehmer (11,12,13) und dazugehörigen Kulissen (14,15,16;17,18;19,20) der Anzahl der vorgesehenen Schließkraftstufen entspricht und daß zwischen jeweils benachbarten Kulissen (14,15,16 und 17,18; 17,18 und 19,20) eine Druckfeder (6,7) eingespannt ist, über welche die Kulissen anschlagfrei in Verbindung stehen, derart, daß für den Fall der kleinstmöglichen Schließkraft und bei Vorgabe von mehr als zwei Schließkraftstufen alle Federn in Reihe geschaltet sind.

4. Zange nach einem der Ansprüche 2 und 3, dadurch gekennzeichnet, daß in den Kulissen (14,15,16;17,18;19,20) Ausnehmungen (22) für den freien axialen Durchgang von nicht in Schaltstellung befindlichen Mitnehmern (11,12,13) vorgesehen sind.

5. Zange nach einem der Ansprüche 2 bis 4, dadurch gekennzeichnet, daß die Druckfedern (6,7) Kulissenteile (17,19) gegen Ringschultern (24,25) zur Anlage bringen, derart, daß die Ringschultern (24,25) in Ruhestellung befindliche Kulissen gegen Bewegung proximalwärts sichern.

6. Zange nach einem der Ansprüche 2 bis 5, dadurch gekennzeichnet, daß die Betätigungsstange (3) proximalseitig einen Schaltring (32) mit einer relativ zu einem feststehenden Teil (39) verdrehbaren Ringfeder (34) aufweist, die mit einer Nase (35) je nach einzustellender Schließkraft in eine von mehreren Ausnehmungen (36,37,38) des feststehenden Teiles (39) eingreift.

7. Zange nach Anspruch 6, dadurch gekennzeichnet, daß die Ringfeder (34) mit einem Stift (40) am Schaltring (32) gegen Verdrehen gesichert ist.

8. Zange nach einem der Ansprüche 2 bis 7, dadurch gekennzeichnet, daß die Drehbewegung des Schaltringes (32) auf den verdrehbaren zweiten Stangenteil (9,10) über eine Zylinderhülse (41) erfolgt.

9. Zange nach einem der Ansprüche 2 bis 8, dadurch gekennzeichnet, daß die Betätigungsstangenteile (8,9,10) mit einem Stift (43) axial beweglich verbunden und gegen Radialbewegung gesichert sind.

10. Zange nach einem der Ansprüche 1 bis 9, dadurch gekennzeichnet, daß die aus der Ruhestellung heraus distalwärts verstellte Betätigungsstange (3) gegen Verdrehen gesichert ist.

11. Zange nach Anspruch 10, dadurch gekennzeichnet, daß auf der Betätigungsstange (3) jeweils ein jedem Mitnehmer (11,12,13) zugeordneter Vorsprung (11a,12a,13a) vorgesehen ist, der bei distalwärtiger Verstellung der Betätigungsstange in drehschlüssigen Eingriff mit einer der Ausnehmungen (22) bringbar ist.

## Claims

1. Forceps having two mouth parts (1, 2) for gripping and holding tissue or the like, in which at least one mouth part (1) can be pivoted relative to the other (2) by adjusting an actuating rod (3) by means of a handle (4), and the closing position of the mouth parts (1, 2) and the handle (4) can be fixed by means of a catch (5), characterised in that the closing force of the mouth parts (1, 2) of the forceps can be adjusted to different values, wherein the force exerted on the actuating rod (3) via the handle (4) is transferred directly to the movable mouth part (1) when setting the maximum closing force and indirectly when setting a further lower closing force via at least one spring (6, 7).

2. Forceps according to claim 1, characterised in that the actuating rod (3) comprises a distal first rod part (8), a second and third rod part (9, 10) extending proximally from the latter, in that the first rod part (8) is directly connected to the movable mouth part (1) and the third rod part (10) is connected to handle (4) via spring clip (31), in that the second rod part (9) can be twisted relative to the first (8) for the purpose of setting the closing force stages and is provided with drivers (11, 12, 13) arranged at an axial distance from one another and offset at angles to one another and which cooperate with axially movable connecting members (14, 15, 16; 17, 18; 19, 20) such that for a preset rotary position of the second rod part (9, 10), only one driver (11) engages with a first connecting member (14, 15, 16) rigidly connected to the first rod part (8), whereas for a further preset rotary position, a further driver (12 or 13) engages with the connecting member (17, 18 or 19, 20) assigned to the latter and transfers the actuating force to the first rod part (8) via this connecting member and at least one compression spring (6, 7).

3. Forceps according to claim 2, characterised in that the number of drivers (11, 12, 13) and associated connecting members (14, 15, 16; 17, 18; 19, 20) corresponds to the number of closing force stages provided, and in that a compression spring (6, 7) is clamped between particular neighbouring connecting members (14, 15, 16 and 17, 18; 17, 18 and 19, 20), by means of which compression spring (6, 7) the connecting members are connected without stops such that all springs are connected in series for the case of as small as possible a closing force and when presetting more than two closing force stages.

4. Forceps according to one of claims 2 and 3, characterised in that recesses (22) for free axial passage of drivers (11, 12, 13) not situated in a controller position are provided in connecting members (14, 15, 16; 17, 18; 19, 20).

5. Forceps according to one of claims 2 to 4, characterised in that compression springs (6, 7) bring connecting member parts (17, 19) to rest against annular shoulders (24, 25), such that annular shoulders (24, 25) secure connecting members situated in the rest position from movement in the proximal direction.

6. Forceps according to one of claims 2 to 5, characterised in that actuating rod (3) has a controller ring (32) on the proximal side having an annular spring (34) which can be twisted relative to a fixed part (39) and which engages with a projection (35) in one of several recesses (36, 37, 38) of fixed part (39) depending on the closing force to be set.

7. Forceps according to claim 6, characterised in that the annular spring (34) is secured against twisting using a pin (40) on the controller ring (32).

8. Forceps according to one of claims 2 to 7, characterised in that the rotary movement of the controller ring (32) on the twistable second rod part (9, 10) takes place via a cylindrical sleeve (41).

9. Forceps according to one of claims 2 to 8, characterised in that the actuating rod parts (8, 9, 10) are connected to be axially movable by means of a pin (43) and secured against radial movement.

10. Forceps according to one of claims 1 to 9, characterised in that the actuating rod (3) adjusted from the rest position in the distal direction is secured against twisting.

11. Forceps according to claim 10, characterised in that in each case a projection (11a, 12a, 13a) assigned to each driver (11, 12, 13), and which can be brought into rotary-positive engagement with one of the recesses (22) during distal adjustment of the actuating rod, is provided on actuating rod (3).

## Revendications

1. Pince comportant deux éléments de mâchoire (1,2) servant à saisir et tenir un tissu ou analogue, dans laquelle au moins un élément de mâchoire (1) peut pivoter par rapport à l'autre (2) par déplacement d'une tige d'actionnement (3) à l'aide d'une poignée (4) et la position fermée des éléments de mâchoire (1,2) et de la poignée (4) peut être fixée par un dispositif d'arrêt (5), caractérisée en ce que la force de serrage des éléments de mâchoire (1,2) de la pince est réglable sur différentes valeurs, la force exercée par l'intermédiaire de la poignée (4) sur la tige d'actionnement (3) étant transmise à l'élément de mâchoire mobile (1) directement, dans le cas du réglage de la force de serrage maximale, et indirectement, dans le cas du réglage d'une autre force de serrage, plus faible, par l'intermédiaire d'au moins un ressort (6,7).

2. Pince selon la revendication 1, caractérisée en ce que la tige d'actionnement (3) est constituée par un premier élément distal (8) et par des second et troisième éléments (9,10), qui s'étendent vers le côté proximal à partir du premier élément de la tige, en ce que le premier élément (8) de la tige est relié directement à l'élément de mâchoire mobile (1) et le troisième élément (10) de la tige est relié à la poignée (4) par l'intermédiaire de ressorts en forme d'étrier (31), en ce que le second élément (9) de la tige peut tourner par rapport au premier (8) en vue du réglage des paliers de la force de serrage et comporte des organes d'entraînement (11,12,13), qui sont disposés à distance l'un de l'autre en direction axiale et sont décalés angulairement les uns par rapport aux autres, et qui coopèrent avec des coulisses déplaçables axialement (14,15,16; 17,18; 19,20), de telle sorte que, dans une position angulaire prédéterminée du second élément (9,10) de la tige , un seul organe d'entraînement (11) est en prise avec une première coulisse (14,15,16) reliée de façon fixe au premier élément (8) de la tige, tandis que, dans une autre position angulaire prédéterminée, un autre organe d'entraînement (12 ou 13) vient en prise avec la coulisse (17, 18 ou 19, 20) associée à ce dernier, et transmet la force d'actionnement au premier élément (8) de la tige par l'intermédiaire de cette coulisse et d'au moins un ressort de compression (6,7).

3. Pince selon la revendication 2, caractérisée en ce que le nombre des organes d'entraînement (11,12,13) et des coulisses (14,15,16; 17,18; 19,20), qui leur sont associées, correspond au nombre des paliers prévus de la force de serrage et en ce qu'entre des coulisses respectivement voisines (14,15,16, et 17, 18; 17,18 et 19,20), est inséré chaque fois un ressort de compression (6,7), au moyen duquel les coulisses sont reliées sans venir en butée de telle sorte que, dans le cas de la force de serrage la plus faible possible et dans le cas de la prédétermination de plus de deux paliers de la force de serrage, tous les ressorts sont montés en série.

4. Pince selon l'une des revendications 2 et 3, caractérisée en ce que, dans les coulisses (14,15,16; 17,18; 19,20), sont prévus des évidements (22) permettant le passage axial libre d'organes d'entraînement (11,12,13) non situés dans la position de commutation.

5. Pince selon l'une des revendications 2 à 4, caractérisée en ce que les ressorts de compression (6,7) appliquent des éléments de coulisse (17,19) contre des épaulements annulaires (24,25), de telle sorte que les épaulements annulaires (24,25) bloquent des coulisses situées en position de repos, contre tout déplacement vers le côté proximal.

6. Pince selon l'une des revendications 2 à 5, caractérisée en ce que la tige d'actionnement (3) comporte, côté proximal, une bague de commutation (32) avec un ressort annulaire (34), qui peut tourner par rapport à un élément fixe (39) et qui s'engage par un bec (35), en fonction de la force de serrage devant être réglée, dans un évidement d'un ensemble de plusieurs évidements (36,37,38) de l'élément fixe (39).

7. Pince selon la revendication 6, caractérisée en ce que le ressort annulaire (34) est bloqué en rotation sur la bague de commutation (32), par une goupille (40).

8. Pince selon l'une des revendications 2 à 7, caractérisée en ce que le mouvement de rotation de la bague de commutation (32) sur le second élément rotatif (9,10) de la tige s'effectue par l'intermédiaire d'une douille cylindrique (41).

9. Pince selon l'une des revendications 2 à 8, caractérisée en ce que les éléments (8,9,10) de la tige d'actionnement sont reliés, avec une possibilité de déplacement axial, par une tige (43), et sont bloqués contre un déplacement radial.

10. Pince selon l'une des revendications 1 à 9, caractérisée en ce que la tige d'actionnement (3), qui est déplacée à partir de la position de repos vers le côté distal, est bloquée en rotation.

11. Pince selon la revendication 10, caractérisée en ce que sur la tige d'actionnement (3), sont prévues des saillies (11a, 12a, 13a), dont chacune est associée à un organe d'entraînement respectif (11,12,13) et peut, dans le cas d'un déplacement vers le côté distal de la tige d'actionnement, être amenée en prise, selon une liaison d'entraînement en rotation, dans l'un des évidements (22).
